# EUROPEAN PATENT SPECIFICATION

(11) **EP 2 373 327 B1**
(45) Date of publication and mention of the grant of the patent: **13.04.2016**
(21) Application number: 08878774.2
(22) Date of filing: 24.12.2008
(51) Int. Cl.: A61K 31/7088, C12N 15/113, A61K 39/395, G01N 33/50, C12Q 1/68

(54) **COMPOSITION COMPRISING EXPRESSION OR ACTIVITY INHIBITORS OF NINJURIN 1 FOR THE PREVENTION AND TREATMENT OF INFLAMMATORY DISEASE**
ZUSAMMENSETZUNG MIT EXPRESSIONS- ODER AKTIVITÄTSHEMMERN VON NINJURIN 1 ZUR PRÄVENTION UND BEHANDLUNG VON ENTZÜNDLICHEN ERKRANKUNGEN
COMPOSITION COMPRENANT DES INHIBITEURS DE L'EXPRESSION OU DE L'ACTIVITÉ DE LA NINJURINE 1 POUR LA PRÉVENTION ET LE TRAITEMENT D'UNE MALADIE INFLAMMATOIRE

(30) Priority: 09.12.2008 KR 20080124555
(43) Date of publication of application: 12.10.2011
(73) Proprietor: SNU R&DB Foundation, Seoul 151-742 (KR)
(72) Inventor: KIM, Kyu-Won, Seoul 151-742 (KR); LEE, Hyo-Jong, Seoul 153-030 (KR)
(74) Representative: Mai, Dörr, Besier
(86) International application number: PCT/KR2008/007629
(87) International publication number: WO 2010/067915

(56) References cited:
- KR-A- 20020 003 468
- US-B2- 7 244 571
- IGAL IFERGAN ET AL: "Role of ninjurin-1 in the migration of myeloid cells to central nervous system inflammatory lesions", ANNALS OF NEUROLOGY, vol. 70, no. 5, 1 November 2011 (2011-11-01), pages 751-763, XP55027992, ISSN: 0364-5134, DOI: 10.1002/ana.22519
- TOYAMA T ET AL: "Ninjurin1 increases p21 expression and induces cellular senescence in human hepatoma cells", JOURNAL OF HEPATOLOGY, MUNKSGAARD INTERNATIONAL PUBLISHERS, COPENHAGEN, DK, vol. 41, no. 4, 1 October 2004 (2004-10-01), pages 637-643, XP004586584, ISSN: 0168-8278, DOI: 10.1016/J.JHEP.2004.06.027
- MANABU KOIKE ET AL: "Characterization of Ninjurin and TSC22 induction after X-irradiation of normal human skin cells", THE JOURNAL OF DERMATOLOGY, vol. 35, no. 1, 1 January 2008 (2008-01-01), pages 6-17, XP55027987, ISSN: 0385-2407, DOI: 10.1111/j.1346-8138.2007.00404.x-i1
- KIM K. ET AL: 'Role of Nijurin1 in the regression of hyaloid vasculature' SYMPOSIUM OF THE KOREAN SOCIETY OF CARDIOLOGY BASIC SCIENCE RESEARCH July 2008, page 2
- ARAKI T. ET AL: 'Mechanism of homophilic binding mediated by Ninjurin, a novel widely expressed adhesion molecule' J. BIOL. CHEM vol. 272, no. 34, 1997, pages 21373 - 21380, XP008140476
- ARAKI T. ET AL: 'Ninjurin, a novel adhesion molecule, is induced by nerve injury and promotes axonal growth' NEURON vol. 17, 1996, pages 353 - 361, XP008140130

<110> SNU R6DB FOUNDATION
<120> Composition comprising expression or activity inhibitors of Ninjurin1 for the prevention and treatment of inflammatory disease
<130> 8fpo-12-10/PCT
<150> KR10-2008-0124555
   <151> 2008-12-09
<160> 17
<170> KopatentIn 1.71
<210> 1
   <211> 152
   <212> PRT
   <213> Homo sapiens
<400> 1
   Met Asp Ser Gly Thr Glu Glu Tyr Glu Leu Asn Gly Gly Leu Pro Pro
    1               5                  10                  15
   Gly Thr Pro Gly Ser Pro Asp Ala Ser Pro Ala Arg Trp Gly Trp Arg
               20                  25                  30
   His Gly Pro Ile Asn Val Asn His Tyr Ala Ser Lys Lys Ser Ala Ala
           35                  40                  45
   Glu Ser Met Leu Asp Ile Ala Leu Leu Met Ala Asn Ala Ser Gln Leu
       50                  55                  60
   Lys Ala Val Val Glu Gln Gly Pro Ser Phe Ala Phe Tyr Val Pro Leu
   65                  70                  75                  80
   Val Val Leu Ile Ser Ile Ser Leu Val Leu Gln Ile Gly Val Gly Val
                   85                  90                  95
   Leu Leu Ile Phe Leu Val Lys Tyr Asp Leu Asn Asn Pro Asp Lys His
               100                 105                 110
   Ala Lys Leu Asp Phe Leu Asn Asn Leu Ala Thr Gly Leu Val Phe Ile
           115                 120                 125
   Ile Val Val Val Asn Ile Phe Ile Thr Ala Phe Gly Val Gln Lys Pro
       130                 135                 140
   Leu Met Asp Met Ala Pro Gln Gln
   145                 150
<210> 2
   <211> 29
   <212> DNA
   <213> Artificial Sequence
<220>
   <223> primer 1 of mouse Ninjurin1 cDNA
<400> 2
   gggaattcca tggagtcggg cactgagga 29
<210> 3
   <211> 31
   <212> DNA
   <213> Artificial Sequence
<220>
   <223> primer 2 of mouse Ninjurin1 cDNA
<400> 3
   ctcctcgagt tctactgccg gggcgccacg t 31
<210> 4
   <211> 20
   <212> DNA
   <213> Artificial Sequence
<220>
   <223> forward primer of Gapdh
<400> 4
   accacagtcc atgccatcac 20
<210> 5
   <211> 20
   <212> DNA
   <213> Artificial Sequence
<220>
   <223> reverse primer of Gapdh
<400> 5
   tccaccaccc tgttgctgta 20
<210> 6
   <211> 17
   <212> DNA
   <213> Artificial Sequence
<220>
   <223> forward primer of Ninjurin1
<400> 6
   gagtcgggca ctgagga 17
<210> 7
   <211> 18
   <212> DNA
   <213> Artificial Sequence
<220>
   <223> reverse primer of Ninjurin1
<400> 7
   gttgcagggg tctggtca 18
<210> 8
   <211> 20
   <212> DNA
   <213> Artificial Sequence
<220>
   <223> forward primer of Ang1
<400> 8
   aggcttggtt tctcgtcaga 20
<210> 9
   <211> 20
   <212> DNA
   <213> Artificial Sequence
<220>
   <223> reverse primer of Ang1
<400> 9
   tctgcacagt ctcgaaatgg 20
<210> 10
   <211> 23
   <212> DNA
   <213> Artificial Sequence
<220>
   <223> forward primer of Ang2
<400> 10
   gctgctggtt tattactgaa gaa 23
<210> 11
   <211> 22
   <212> DNA
   <213> Artificial Sequence
<220>
   <223> reverse primer of Ang2
<400> 11
   tcaggtggac tgggatgttt ag 22
<210> 12
   <211> 22
   <212> DNA
   <213> Artificial Sequence
<220>
   <223> forward primer of Wnt7b
<400> 12
   aagaactccg agtagggagt cg 22
<210> 13
   <211> 22
   <212> DNA
   <213> Artificial Sequence
<220>
   <223> reverse primer of Wnt7b
<400> 13
   tgcgttgtac ttctccttga gc 22
<210> 14
   <211> 20
   <212> DNA
   <213> Artificial Sequence
<220>
   <223> forward primer of Wnt7b 2nd round
<400> 14
   ccgagtaggg agtcgagagg 20
<210> 15
   <211> 22
   <212> DNA
   <213> Artificial Sequence
<220>
   <223> reverse primer of Wnt7b 2nd round
<400> 15
   cacaccgtga cacttacatt cc 22
<210> 16
   <211> 20
   <212> DNA
   <213> Artificial Sequence
<220>
   <223> forward primer of Ang2
<400> 16
   tgtgatcttg tcttggccgc 20
<210> 17
   <211> 21
   <212> DNA
   <213> Artificial Sequence
<220>
   <223> reverse primer of Ang2
<400> 17
   agagggagtg ttccaagaag c 21

## Description

### [Technical Field]

The present invention relates to a composition comprising a Ninjurin 1 expression or activity inhibitor for the prevention and treatment of inflammatory disease. Particularly, Ninjurin 1 protein specifically expressed in macrophages around blood vessels interacts with vascular endothelial cells to induce apoptosis of the cells via Wnt7b-Ang signal transduction pathway, increases iNOS expression and promotes NO generation to induce inflammation at last. And the present invention relates to a composition comprising such a Ninjurin 1 expression or activity inhibitor as an active ingredient for the prevention and treatment of inflammatory disease.

### [Background Art]

Ninjurin 1 was first reported by Toshiyuki Araki et al in 1996 (Araki, T. & Milbrandt, J., Neuron 17, 353-361, 1996). It was found during the screening of a gene up-regulated in Schwann cells after damage caused by transection or crush in sciatic nerve.

Phylogenetic tree was made with proteins having homology with Ninjurin based on protein information in GeneBank (Zhang, S. et al., Genes & development 20, 1899-1910, 2006). It was reported that vertebrate have two Ninjurin proteins, Ninjurin 1 and Ninjurin 2. In fact, Ninjurin 1 and Ninjurin 2 were found in vertebrate such as human, mouse and rat. Ninjurin identified in invertebrate is classified into three types, A, B, and C, and specifically identified in drosophila, mosquito, and so on. Human Ninjurin 1 shows 90% homology with mouse Ninjurin 1 (Chadwick, B.P. et al., Genomics 47, 58-63, 1998). In the meantime, human Ninjurin 1 shows 55% homology with human Ninjurin 2 (Araki, T. & Milbrandt, J., J Neurosci 20, 187-195, 2000).

Human Ninjurin 1 is located at chromosome 9q22 and is composed of 152 amino acids. Mouse Ninjurin 1, in the meantime, is located at chromosome 13 and is composed of 152 amino acids. Two transmembrane domains are predicted in the amino acid sequence of Ninjurin 1. It was also confirmed by experiments that Ninjurin 1 is the protein located in cell membrane (Araki, T. & Milbrandt, J., Neuron 17, 353-361, 1996). Accordingly, it can be predicted that N-terminal region of Ninjurin 1 is stretched long out of cell.

Ninjurin 1 is expressed in diverse tissues. For example, it is expressed at RNA level in the heart, brain, placenta, lung, liver, SK. Muscle, kidney, pancreas, spleen, thymus, prostate, testis, ovary, small int., colon, blood, adrenal gland and dorsal root ganglia (DRG). It is expressed at protein level in the liver, kidney, thymus, uterus, adrenal gland, retina and dorsal root ganglia (Araki, T. et al., The Journal of biological chemistry 272, 21373-21380, 1997).

The functions of Ninjurin 1 known so far are in relation to 1) cell adhesion, 2) neurite outgrowth, 3) cellular senescence, and 4) cancer.

Particularly, regarding the function of Ninjurin 1 in relation to cell adhesion 1), it was reported via cell aggregation experiment performed with Jurkat T cell leukemia that Ninjurin 1 increased aggregation among cells (Araki, T. & Milbrandt, J., Neuron 17, 353-361, 1996). For the cell adhesion, polymerization of actin filaments, oxidative phosphorylation, divalent cation and proper pH (pH 7-11) are required (Araki, T. et al., The Journal of biological chemistry 272, 21373-21380, 1997). In the study using drosophila, Ninjurin A protein digested with MMP1 (matrix metalloproteinase 1) acted as a signal molecule to inhibit cell adhesion (Zhang, S. et al., Genes & development 20, 1899-1910, 2006).

To investigate the function of Ninjurin 1 in relation to neurite outgrowth 2), CHO cells were monolayer-cultured, on which DRG neurite cells were cultured. When CHO cells over-expressing Ninjurin 1 were used, neurite cell proliferation was increased (Araki, T. & Milbrandt, J., Neuron 17, 353-361, 1996). When the amino acid sequence ranging from the 26^{th} to the 37^{th} residue of Ninjurin 1 protein was modified, neurite cell proliferation was inhibited (Zhang, S. et al., Genes & development 20, 1899-1910, 2006). When DRG neurite cells and skin-derived fibroblast-like cells (FLCs) were co-cultured, when Ninjurin 1 was expressed, neurite cells were being proliferated but when Ninjurin 1 was inhibited by an antibody not to be functioning, neurite cell proliferation was inhibited (Jerregard, H. et al., Journal of neurocytology 30, 327-336, 2001).

Regarding cellular senescence 3), when Ninjurin 1 was over-expressed, cell cycle was arrested in G1 stage after p21^{WAF1/Cipl} transcription, resulting in a significant inhibition of cell proliferation. Besides, when Ninjurin 1 was over-expressed, senescence-associated β-galactosidase activity and autofluorescence pigment were increased. Ninjurin 1 is also up-regulated in hepatocellular carcinoma tissue, suggesting that Ninjurin 1 might be involved in cellular senescence which is the target of anti-cancer treatment (Toyama, T. et al., Journal of hepatology 41, 637-643, 2004).

In studies of Ninjurin 1 in relation to cancer 4), Ninjurin 1 was confirmed to be up-regulated in hepatocellular carcinoma including virus infection in the liver or cirrhosis (Kim, J.W. et al., Molecules and cells 11, 151-157, 2001). Ninjurin 1 was also increased in acute lymphocytic leukemia. Ninjurin 1 was directly increased during the screening of a gene regulated by the tumor suppressing protein p53 using microarray (Kannan, K. et al., Oncogene 20, 2225-2234, 2001) .Ninjurin 1 was found to be strongly induced in Keratinocytes after x-ray irradiation (Koike, M. et al., Journal of Dermatology 35, 6-17, 2008).

However, it has not been disclosed yet whether Ninjurin 1 is involved in the functions of macrophages, vascular decrease and inflammation induction, but Ninjurin 1 was identified as a marker of inflammation (Hakanarson, H. et al., US7,244,571-B2).

So, the present inventors have been studied on the involvement and mechanism of Ninjurin 1 in relation to macrophages, during which the inventors confirmed that Ninjurin 1 was expressed specifically in macrophages around blood vessels, increased cell-matrix and cell-cell adhesion, increased Wnt7b (Wingless-type MMTV integration site family, member 7B) and Ang2 (angiopoietin-2) expressions, and accelerated apoptosis of vascular endothelial cells (VECs) by reducing Ang1 (angiopoietin-1) expression. Further, the present inventors completed this invention by confirming that Ninjurin 1 was up-regulated when inflammation was induced by LPS *in vivo* and *in vitro* and increased iNOS expression and NO generation and accordingly confirming that Ninjurin 1 increased the activity of macrophages to induce inflammation.

### [Disclosure]

### [Technical problem]

It is an object of the present invention to provide a method for preventing and treating inflammatory disease caused by over-activation of macrophages containing the step of inhibiting the expression or activity of Ninjurin 1 protein.

### [Technical Solution]

To achieve the above object, the present invention provides a composition comprising a Ninjurin 1 protein expression or activity inhibitor for the prevention and treatment of inflammatory disease.

The composition of the present invention can be used for treating inflammatory disease containing the step of administering a pharmaceutically effective dose of the said composition to a subject with inflammatory disease.

The composition of the present invention can be used for preventing inflammatory disease by administering a pharmaceutically effective dose of the said composition to a subject.

The present invention also provides a use of a Ninjurin 1 expression or accivity inhibitor for the preparation of a composition for the prevention and treatment of inflammatory disease.

In addition, the present invention provides a screening method of a preventive and therapeutic agent for inflammatory disease comprising the following steps:
1) treating samples to a cell line expressing Ninjurin-1 protein;
2) measuring the expression of Ninjurin 1 protein in the cell line; and
3) selecting a sample that inhibited the expression of Ninjurin 1 protein, compared with the expression level in the control.

### Hereinafter, the present invention is described in detail.

The present invention provides a composition comprising a Ninjurir 1 protein expression or activity inhibitor for the prevention and treatment of inflammatory disease according to claim 1.

The said Ninjurin 1 protein preferably has the amino acid sequence represented by SEQ. ID. NO: 1, but not always limited thereto.

The Ninjurin 1 protein expression inhibitor is selected from the group consisting of an antisense nucleotide complementarily binding to Ninjurin 1 mRNA, short interfering RNA and short hairpin RNA.

The Ninjurin 1 protein activity inhibitor is an antibody.

The inflammatory disease herein is preferably selected from the group consisting of rheumatic arthritis, inflammatory bowel disease, ankylosing spondylitis, psoriasis, atherosclerosis and multiple sclerosis, and any inflammatory disease caused by over-activation of macrophages can be included.

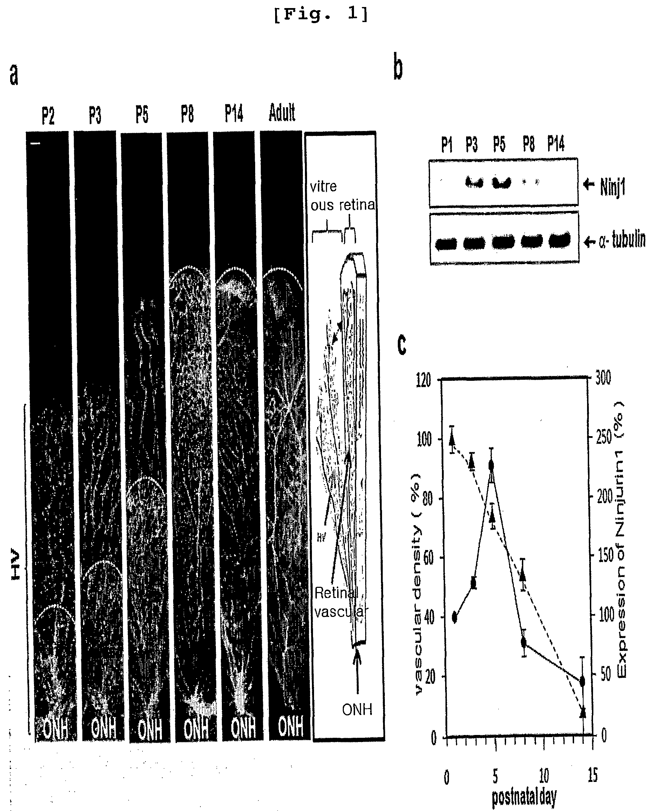

In this invention, to investigate the relation of Ninjurin 1 with macrophages, mice at different days after birth were prepared and Ninjurin 1 protein expression and location where Ninjurin 1 protein was expressed particularly in oculus were investigated under immunofluorescece microscope and by Western blotting during ocular development stage. As a result, Ninjurin 1 was largely expressed in the early ocular development stage and specifically in macrophages around blood vessels (see Figures 1-4). The above result indicates that Ninjurin 1 is expressed specifically in macrophages and is involved in the interaction of macrophages with blood vessel cells.

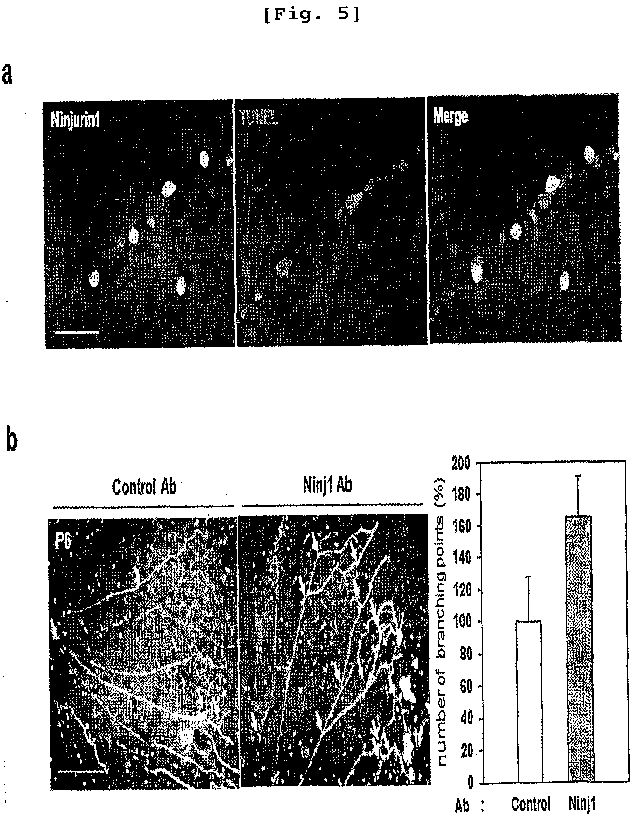

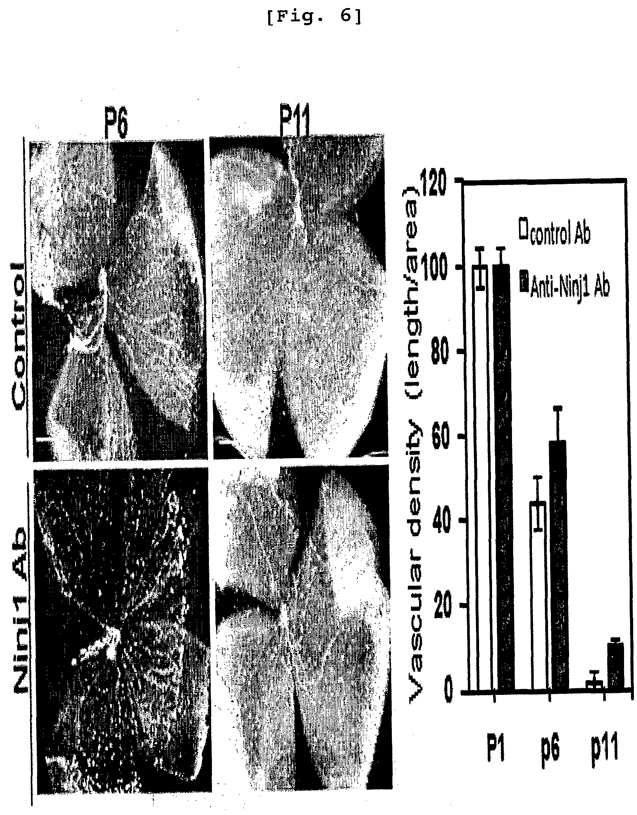

In this invention, to investigate the effect of Ninjurin 1 on vascular endothelial cells, oculus was extracted respectively from a mouse at some days after birth and a mouse where Ninjurin was neutralized by a Ninjurin 1 antibody. Then, the locations and amounts of macrophages and vascular endothelial cells in the oculus were observed under immunofluorescece microscope. As a result, macrophages expressing Ninjurin 1 were adhered on vascular endothelial cells which were being through apoptosis, and the level of vitreous vascular endothelial cells in the mouse neutralized by a Ninjurin 1 antibody was higher than that in the control (see Figures 5 and 6). The above results indicate that macrophages expressing Ninjurin 1 are involved in apoptosis of vitreous vascular endothelial cells.

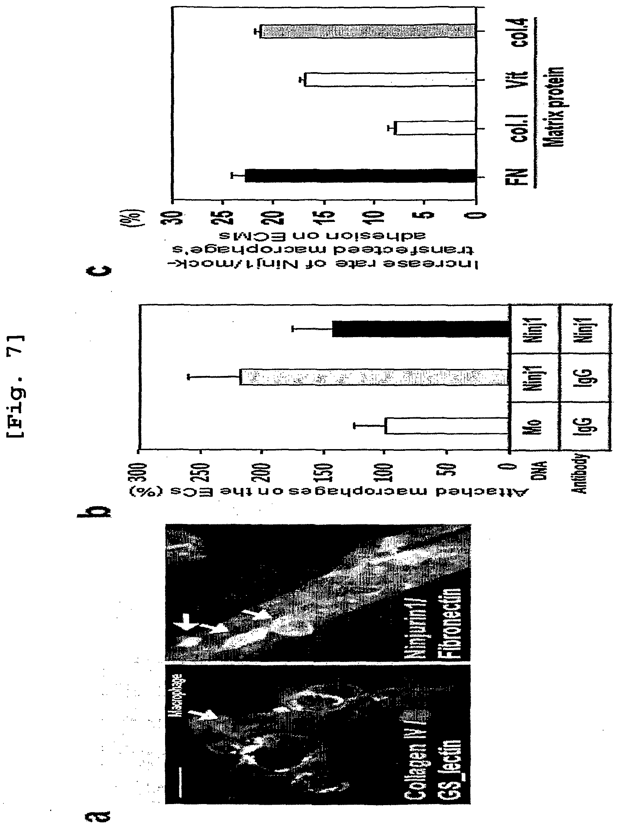

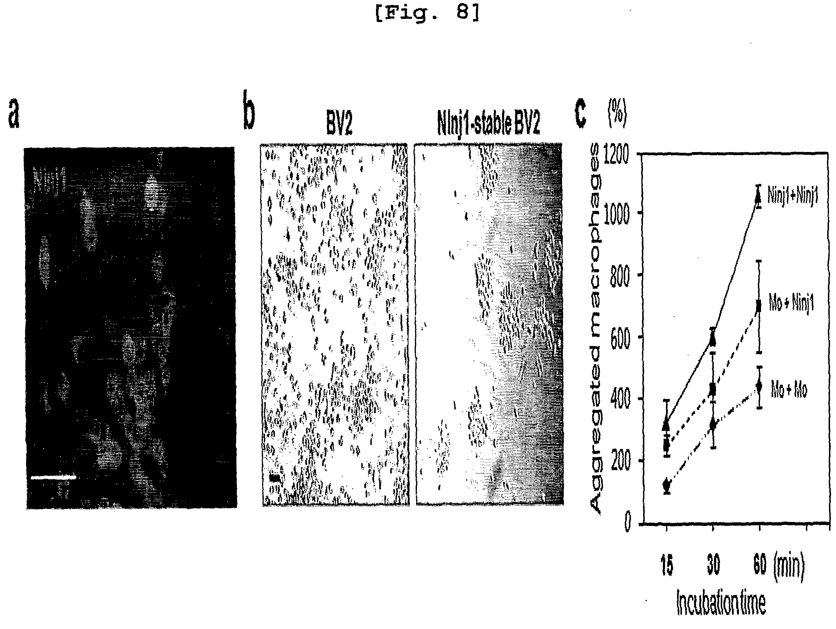

In this invention, to investigate the effect of Ninjurin 1 on cell-cell adhesion and cell-matrix adhesion, cells transformed or not transformed with Ninjurin 1 were used for the experiment examining cell adhesion to different matrixes and coagulation. As a result, over-expression of Ninjurin 1 increased macrophage-vascular endothelial cell adhesion and also increased macrophage binding to such matrixes as collagen, fibronectin and vitronectin, and at the same time increased coagulation near blood vessel cells (see Figures 7 and 8). Therefore, it was confirmed that Ninjurin 1 was involved in cell-cell adhesion and cell-matrix adhesion.

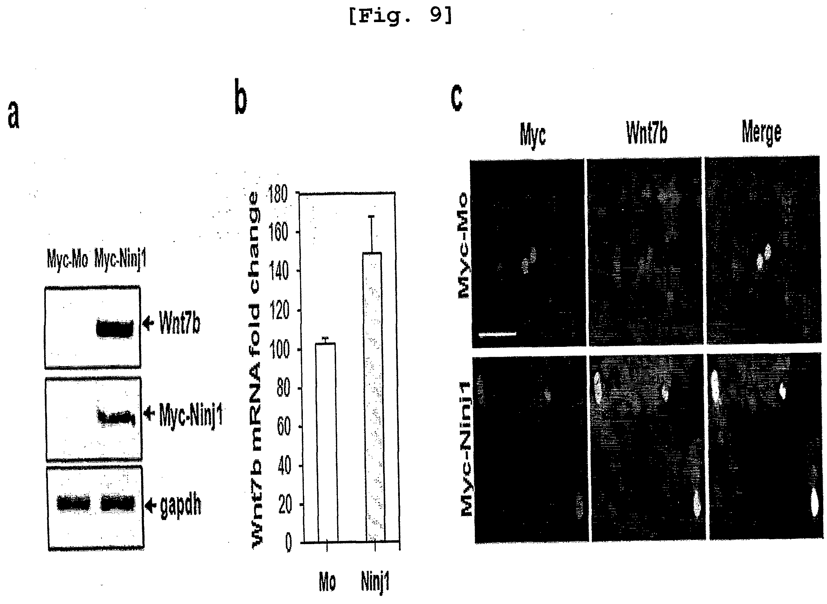

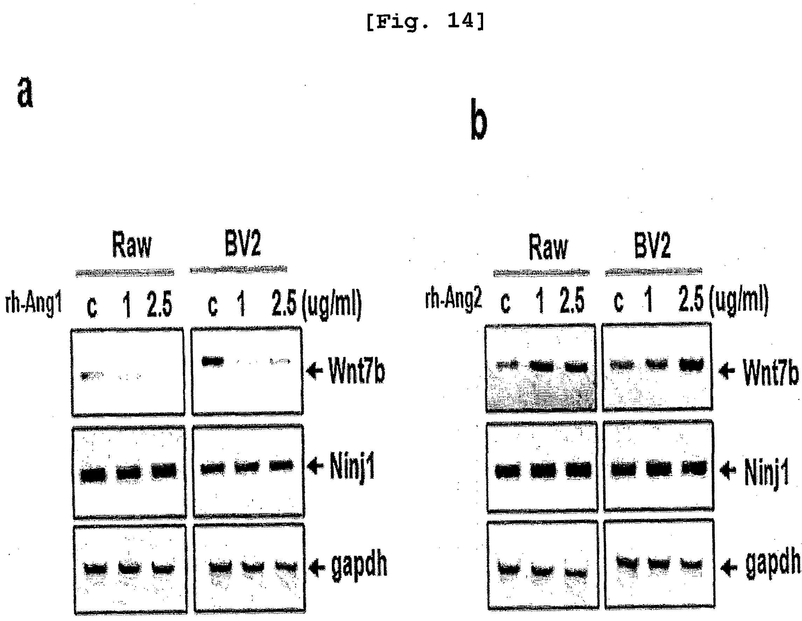

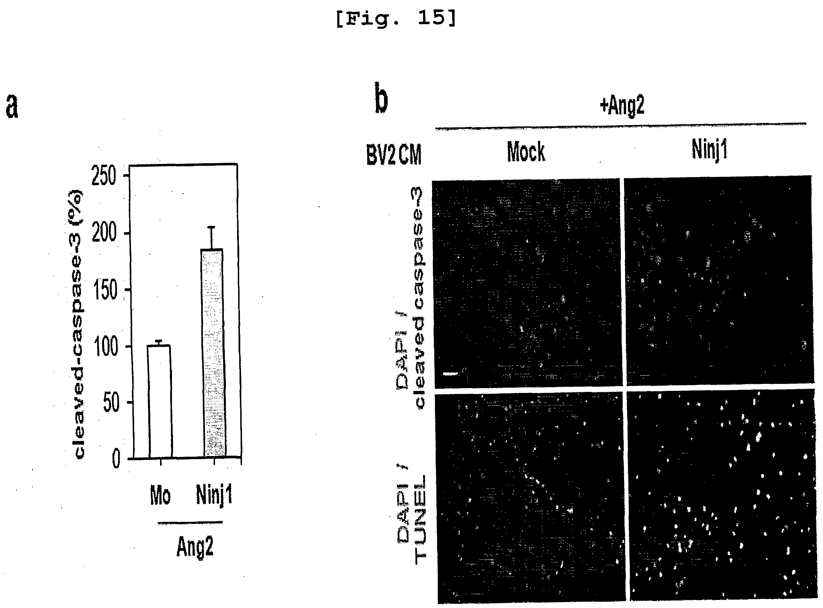

In this invention, to investigate the effect of Ninjurin 1 on Wnt-Ang signal transduction system, expression patterns of Wnt7b, p38, MAPK (p44/p42), JNK (p54/46), Ang1 and Ang2 over the Ninjurin 1 expression patterns were investigated in Ninjurin 1 over-expressing cells and in Ninjurin 1 expression inhibited cells by RT-PCR and real-time PCR. As a result, when Ninjurin 1 expression was increased, expressions of Wnt7b, phosphorylated p38, MAPK (p44/p42) and JNK (p54/46) were also increased (see Figure 9 - Figure 13). To investigate the relation of Ninjurin 1 and Wnt7b with Ang1 and Ang2, intracellular concentrations of Wnt7b and Ninjurin 1 were investigated in the presence of different concentrations of Ang1 or Ang2. As a result, Wnt7b was down-regulated as Ang1 concentration was increased, while Wnt7b was up-regulated as Ang2 concentration was increased (see Figure 14). To investigate the effect of Ninjurin 1 on apoptosis mediated by Ang2, pericytes were treated with the cell culture solution in which Ninjurin 1 was over-expressed in the presence of Ang2, followed by examining digestion by caspase 3 and apoptosis. As a result, over-expression of Ninjurin 1 increased digestion by caspase3 and apoptosis (see Figure 15). In conclusion, Ninjurin 1 increased the expressions of Wnt7b and Ang2 but reduced the expression of Ang1, suggesting that Ninjurin 1 induced apoptosis of vitreous vascular endothelial cells by regulating Wnt-Ang signal transduction system.

That is, Ninjurin 1 is specifically expressed in macrophages around blood vessels, so that it mediates direct interaction between macrophages and vascular endothelial cells and thereby activates Wnt-Ang pathway, resulting in the induction of apoptosis and decrease of blood vessels. Therefore, apoptosis in blood vessel cells can be inhibited by suppressing expression or activation of Ninjurin 1.

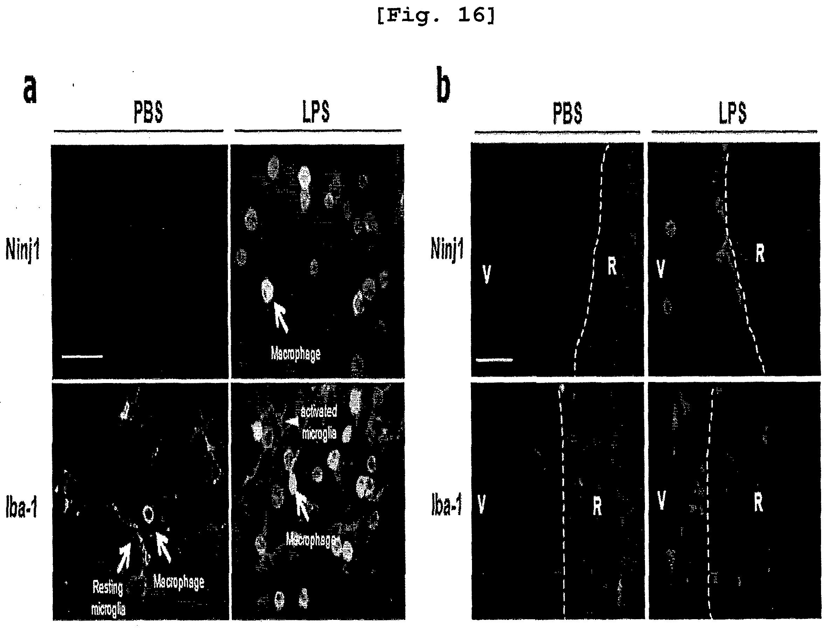

In this invention, to investigate Ninjurin 1's involvement in inflammation reaction, lipopolysaccharide (LPS) was intraperitoneally injected into a rat to induce inflammation. Then, oculus was extracted, followed by observation by immunofluorescence staining. As a result, LPS administration increased the number of macrophages expressing Ninjurin 1 (see Figure 16).

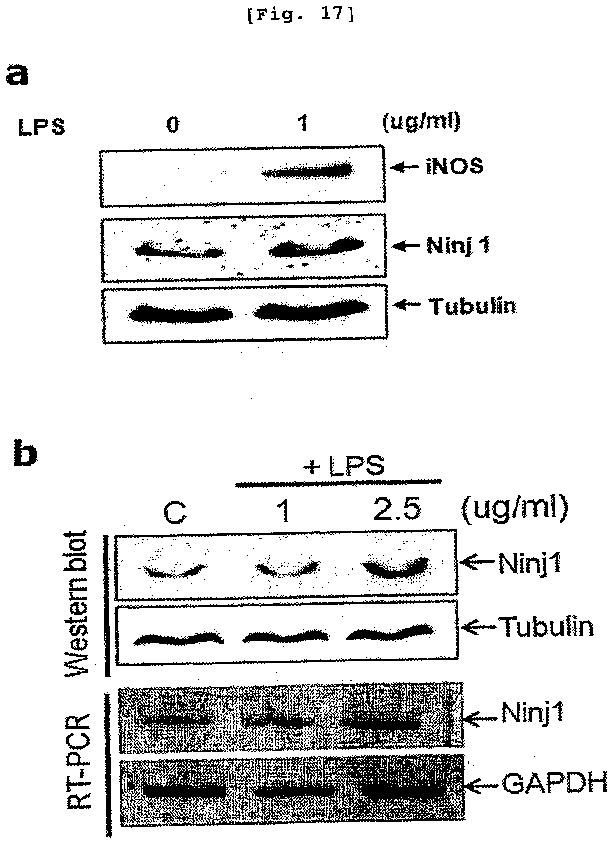

In this invention, cells were treated with LPS to induce inflammation. Then, Ninjurin 1 expression and iNOS expression, the index of inflammation, were investigated and also iNOS expression patterns varying from Ninjurin 1 expression and generation of nitric oxide (NO), the inflammatory mediator, were measured. As a result, as LPS concentration was increased, Ninjurin 1 expression was also increased. And as Ninjurin 1 expression was increased, iNOS expression and NO generation were increased (see Figure 17 - Figure 20). In conclusion, Ninjurin 1 was up-regulated when inflammation was induced and the increase of Ninjurin expression resulted in the increase of iNOS expression and NO generation, suggesting that Ninjurin 1 is involved in inflammation inducing mechanism.

That is, Ninjurin 1 increases iNOS and NO levels to induce inflammation. So, inflammation can be inhibited by suppressing Ninjurin 1 expression or activation.

As explained hereinbefore, Ninjurin 1 increases the activity of macrophages, and over-activation of macrophages causes diverse diseases including rheumatic arthritis, inflammatory bowel disease, ankylosing spondylitis, psoriasis (Morand, E.F. et al., Intern Med J 35, 419-426, 2005), atherosclerosis (Choudhury, R.P., et al., Nat Clin Pract Cardiovasc Med 2, 309-315, 2005), and multiple sclerosis (Minagar, A. et al., J Neurol Sci 202, 13-23, 2002), etc. Therefore, such diseases, particularly inflammatory disease caused by over-activation of macrophages can be prevented and treated by inhibiting Ninjurin 1 expression or activation.

The composition for the prevention and treatment of inflammatory disease comprising a Ninjurin 1 protein expression or activation inhibitor as an active ingredient can include the said active ingredient by 0.0001 - 50 weight% by the total weight of the composition.

The composition of the present invention can include one or more effective ingredients having the same or similar function to the Ninjurin 1 protein expression or activation inhibitor.

The composition of the present invention can include one or more pharmaceutically acceptable carriers such as saline, sterilized water, Ringer's solution, buffered saline, dextrose solution, maltodextrin solution, glycerol, ethanol, liposome and a mixture comprising one or more of those components in addition to the said active ingredient. If necessary, a general additive such as an antioxidant and buffer can be additionally added. The composition of the present invention can be formulated in different forms including aqueous solutions, suspensions and emulsions for injection, pills, capsules, granules or tablets by mixing with diluents, dispersing agents, surfactants, binders and lubricants. A target organ specific antibody or other ligands can be mixed with one of the said carriers to be delivered to the target organ. The composition can further be prepared in suitable forms according to ingredients by following the method represented in Remington's Pharmaceutical Science (the newest edition), Mack Publishing Company, Easton PA.

Nucleotide or nucleic acid used in this invention can be formulated for oral, local, parenteral, intranasal, intravenous, intramuscular, hypodermic, ophthalmic or transdermal administration. It is more preferred to prepare nucleic acid or vector as an injectable formulation. For direct injection, the injectable composition can be mixed with a pharmaceutically acceptable carrier. The composition of the present invention can also include a freeze-dried composition facilitating injection using sterilized isotonic solution or distilled water or saline. Direct injection of nucleic acid into a tumor of a patient brings the effect of focusing the treatment effect on infected tissues, which favors the treatment. Dosage of the nucleic acid can be regulated according to diverse parameters, particularly a gene or a vector, administration method, target disease and required treatment period, etc. in addition, weight, age, gender, health condition, administration tines, administration method, excretion and severity of a disease. The preferable dosage is 0.0001-100 mg/kg per day and more preferably 0.001-10 mg/kg per day, and administration frequency is once a day or preferably a few times a day.

The composition of the present invention can be used for treating inflammatory disease by administering a pharmaceutically effective dose of the said composition to a subject with inflammatory disease.

The composition of the present invention can also be used for preventing inflammatory disease by administering a pharmaceutically effective dose of the said composition to a subject.

The inflammatory disease herein is preferably selected from the group consisting of rheumatic arthritis, inflammatory bowel disease, ankylosing spondylitis, psoriasis, atherosclerosis and multiple sclerosis, but not always limited thereto and any inflammatory disease caused by over-activation of macrophages can be included.

The composition of the present invention can include one or more effective ingredients having the same or similar function to the Ninjurin 1 protein expression or activation inhibitor.

The composition of the present invention can be administered orally or parenterally and be used in general forms of pharmaceutical formulation.

The composition of the present invention can be prepared for oral or parenteral administration by mixing with generally used diluents or excipients such as fillers, extenders, binders, wetting agents, disintegrating agents and surfactant. Formulations for parenteral administration are sterilized aqueous solutions, water-insoluble excipients, suspensions, emulsions, lyophilized preparations, suppositories and injections. Water insoluble excipients and suspensions can contain, in addition to the active compound or compounds, propylene glycol, polyethylene glycol, vegetable oil like olive oil, injectable ester like ethylolate, etc. Suppositories can contain, in addition to the active compound or compounds, witepsol, macrogol, tween 61, cacao butter, laurin butter, glycerogelatin, etc.

The effective dosage of the composition can be determined according to weight, age, gender, health condition, diet, administration frequency, administration method, excretion and severity of a disease. The preferable dosage is 0.0001-100 mg/kg per day and more preferably 0.001-10 mg/kg per day, and administration frequency is once a day or preferably a few times a day.

The composition of the present invention can be administered alone or treated together with surgical operation, hormone therapy, chemo-therapy and biological regulators.

The present invention also provides a use of a Ninjurin 1 expression or activity inhibitor for the preparation of a composition for the prevention and treatment of inflammatory disease according to claim 1.

The said Ninjurin 1 protein preferably has the amino acid sequence represented by SEQ. ID. NO: 1.

The Ninjurin 1 protein expression inhibitor is selected from the group consisting of an antisense nucleotide complementarily binding to Ninjurin 1 mRNA, short interfering RNA and short hairpin RNA.

The Ninjurin 1 protein activity inhibitor is an antibody.

The inflammatory disease herein is preferably selected from the group consisting of rheumatic arthritis, inflammatory bowel disease, ankylosing spondylitis, psoriasis, atherosclerosis and multiple sclerosis, but not always limited thereto and any inflammatory disease caused by over-activation of macrophages can be included.

Ninjurin 1 protein of the present invention is expressed specifically in macrophages around blood vessels, increases cell-cell adhesion and cell-matrix adhesion, increases the expressions of Wnt7b and Ang2, reduces Ang1 expression and accelerates apoptosis of vascular endothelial cells. Ninjurin 1 protein is up-regulated when inflammation is induced, and then it increases iNOS expression and NO generation. Therefore, a Ninjurin 1 protein expression or activation inhibitor can be effectively used as an active ingredient of a composition for the prevention and treatment of inflammatory disease.

In addition, the present invention provides a screening method of a preventive and therapeutic agent for inflammatory disease comprising the following steps:
1) treating samples to a cell line expressing Ninjurin 1 protein;
2) measuring the expression of Ninjurin 1 protein in the cell line; and
3) selecting a sample that inhibited the expression of Ninjurin 1 protein, compared with the expression level in the control.

In this method, the Ninjurin 1 protein of step 1) preferably has the amino acid sequence represented by SEQ. ID. NO: 1.

In this method, the protein expression of step 2) is measured by one of the methods selected from the group consisting of immunofluorescence method, ELISA, Western blotting, and RT-PCR.

In this method, the inflammatory disease of step 2) is preferably selected from the group consisting of rheumatic arthritis, inflammatory bowel disease, ankylosing spondylitis, psoriasis, atherosclerosis and multiple sclerosis.

### [Advantageous Effect

Ninjurin 1 protein of the present invention is expressed specifically in macrophages around blood vessels and then mediated direct interaction between macrophages and vascular endothelial cells, by which it activates Wnt-Ang pathway to induce apoptosis. Ninjurin 1 also increases iNOS expression and NO generation, suggesting that it is directly involved in inflammation reaction. Therefore, the said Ninjurin 1 expression or activation inhibitor can be effectively used as an active ingredient of a composition for the prevention and treatment of inflammatory disease.

### [Description of Drawings]

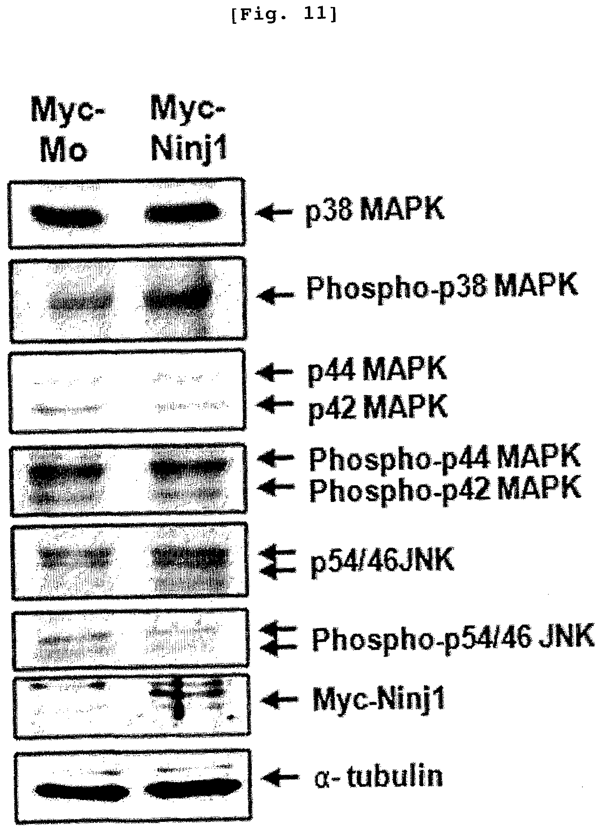

The application of the preferred embodiments of the present invention is best understood with reference to the accompanying drawings, wherein:
Figure 1 is a set of diagrams illustrating the ocular development stage of a mouse and Ninjurin 1 expression over the time: (a) vitreous and incipient retinal vessel stained with GS-lectin (green) of mice at 2 days (P2), at 3 days (P3), at 5 days(P5), at 8 days(P8), and at 14 days (P14) from birth and an adult mouse; (b) graph illustrating Ninjurin 1 protein expression in oculus of those mice examined by Western blotting; (c) graph illustrating blood vessel density (A) and Ninjurin 1 expression (•) over the ocular development in those mice (considering the value at P1 as 100%).

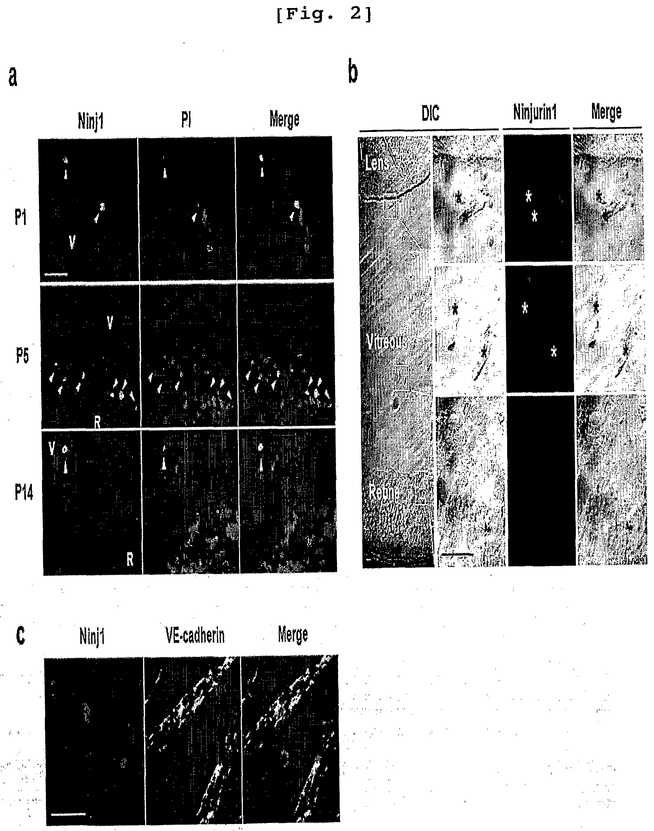

Figure 2 is a set of diagrams illustrating the Ninjurin 1 expression and location in the mouse oculus: (a) diagram showing cellular location where Ninjurin 1 expression is observed in cross-sections of oculus of mice at 1 day (P1), at 5 days (P5) and at 14 days (P14) from birth. At this time, Ninjurin 1 was stained green and nucleus was stained with propidium iodide (PI) (red): V: vitreous, R: retina, white arrow: cells expressing Ninjurin 1. (b) diagram illustrating the DIC microscope imaging of the oculus, in which Ninjurin 1 was stained red; (c) diagram illustrating Ninjurin 1 (red) and VE-cadherin (green) in the oculus extracted from the mouse at 5 days (P5), observed by double immunostaining.

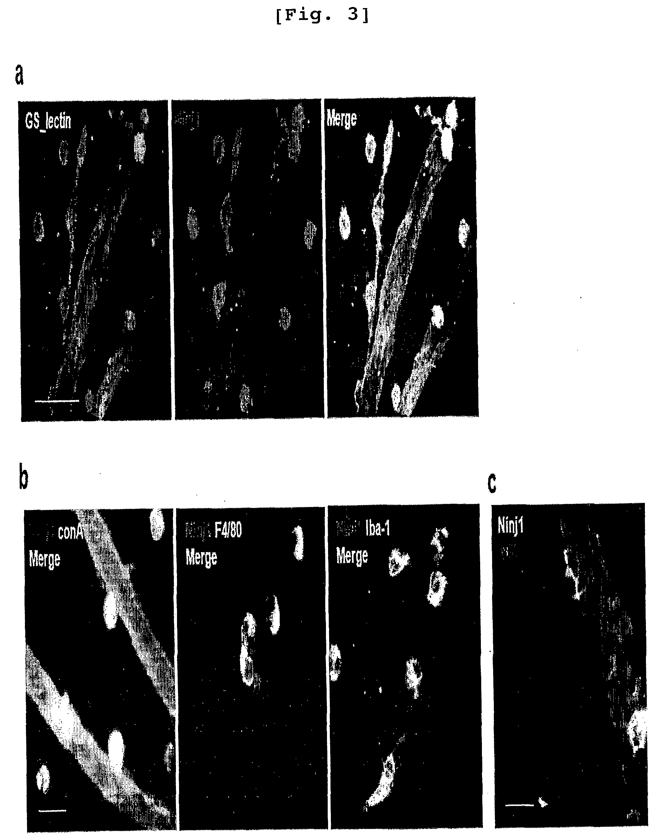

Figure 3 is a set of diagrams illustrating the location of Ninjurin 1 expression around blood vessels in the mouse oculus: (a) diagram illustrating GS-lectin (green) and Ninjurin 1 (red) (if overlapped: yellow) in the oculus of the mouse at 5 days (P5), observed under fluorescent microscope after double immunostaining; (b) diagram illustrating Ninjurin 1 (red) and ConA (green) (if overlapped: yellow), Ninjurin 1 (red) and F4/80 (green) (if overlapped: yellow), and Ninjurin 1 (red) and Iba-1 (green) (if overlapped: yellow) in the oculus, observed under fluorescent microscope after double immunostaining; and (c) diagram illustrating Ninjurin 1 (green) and NG2 (red) (if overlapped: yellow) in the oculus, observed under fluorescent microscope after double immunostaining.

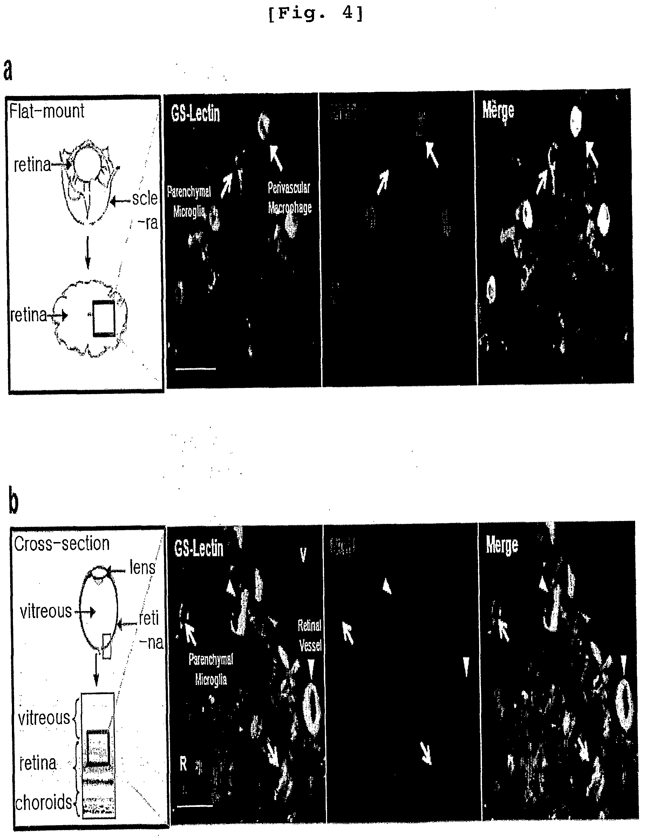

Figure 4 is a set of diagrams illustrating the location of Ninjurin 1 expression and macrophages around blood vessels in the mouse oculus: (a) diagram illustrating Ninjurin 1 (red) and GS-lectin (green) in the oculus of the mouse at 5 days (P5), observed under fluorescent microscope after double immunostaining. At this time, hyaloid vessels were eliminated from the oculus using 5% gelatin. (b) diagram illustrating Ninjurin 1 (red) and parenchymal microglia (green) in the retina, observed under fluorescent microscope after double immunostaining (white arrow: retinal vessel; yellow arrow: parenchymal microglia stretching its arm).
Figure 5 is a set of diagrams illustrating the changes of vascular endothelial cells over Ninjurin 1 expression in the mouse vitreous; (a) diagram illustrating that macrophages expressing Ninjurin 1 in vitreous of the mouse at 8 days (P8) were adhered to vascular endothelial cells being through apoptosis, which was observed under immunofluorescent microscope; and (b) diagram illustrating that vitreous of the mouse at 6 days (P6) neutralized with Ninjurin 1 antibody was stained with GS-lectin (green), followed by observation under fluorescent microscope (yellow arrow: branching point of blood vessel).
Figure 6 is a diagram illustrating the changes of blood vessel density over Ninjurin 1 expression, in which oculus of a mouse at 6 days (P6) belonging to the control and oculus of a mouse at 6 days (P6) neutralized with a Ninjurin 1 antibody were stained with GS-lectin (green), followed by observation under fluorescent microscope.
Figure 7 is a set of diagrams illustrating the cell-matrix adhesion over Ninjurin 1 expression: (a) diagram illustrating Type IV collage (red) and GS-lectin (green), and Ninjurin 1 (red) and fibronectin (green) in the vitreous, observed under fluorescent microscope after double immunostaining; (b) graph illustrating the cell-matrix adhesion in BV2 cells transformed with pCS2+-Ninjurin 1 and in BV2 cells transformed with pCS2+-Mock which were both neutralized by a Ninjurin 1 antibody and control IgG respectively; and (c) graph illustrating the result of investigation of adhesion of BV2 cells transformed with pCS2+-Ninjurin 1 and BV2 cells transformed with pCS2+-Mock to different matrixes (FN: fibronectin; Col. I : type I collagen; Vit: vitronectin; Col.IV: type IV collagen).
Figure 8 is a set of diagrams illustrating the cell-cell adhesion over Ninjurin 1 expression; (a) diagram illustrating that Ninjurin 1 (red) in the vitreous of the mouse at 3 days (P3) was stained, followed by observation under fluorescent microscope; (b) diagram illustrating the coagulation of wild type BV2 cells and BV2 cells (Ninj1-stable BV2) expressing Ninjurin 1 constantly, observed after 2 day culture; and (c) graph illustrating the coagulation of cultured Mock-Ninjurin 1 expressing cell mixture.
Figure 9 is a set of diagrams illustrating the Wnt7b expression over Ninjurin 1 expression: (a) diagram illustrating the result of RT-PCR, in which Wnt7b expressions in BV2 cells expressing Myc-mock and BV2 cells expressing Myc-Ninjurin 1 were shown; (b) diagram illustrating the result of real-time PCR; and (c) diagram illustrating the result of immunostaining examining the expressions of Wnt7b (green) and c-Myc (red).

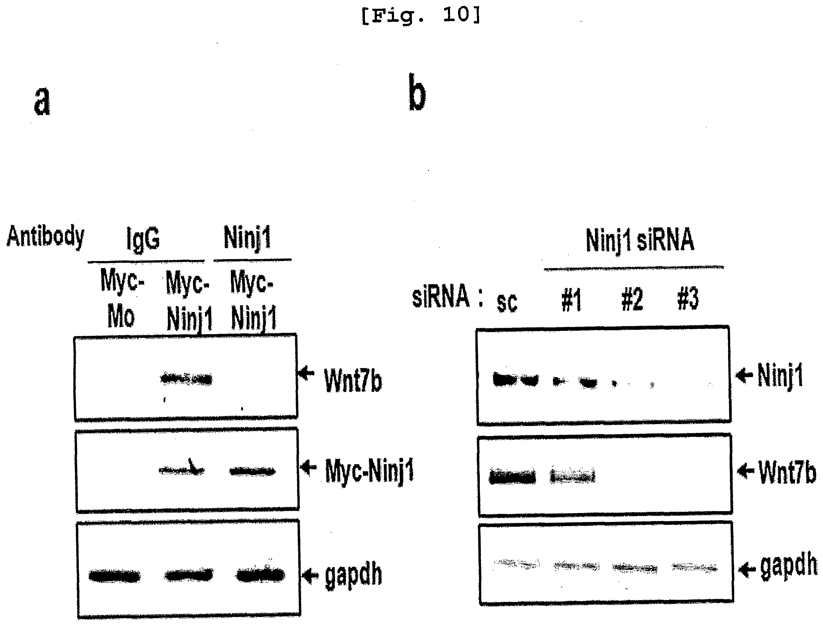

Figure 10 is a set of diagrams illustrating the Wnt7b expression over Ninjurin 1 expression: (a) diagram illustrating the result of RT-PCR investigating Wnt7b expression in BV2 cells expressing Myc-Ninjurin 1 neutralized with Ninjurin 1 antibody; and (b) diagram illustrating the result of RT-PCR examining Wnt7b expression in BV2 cells in which Ninjurin 1 was suppressed by siRNA.
. Figure 11 is a diagram illustrating the expressions of p38, MAPK (p44/p42) and JNK (p54/46) over Ninjurin 1 expression. This picture shows the result of Western blotting with cell lysate of BV2 cells expressing Myc-mock and BV2 cells expressing Myc-Ninjurin 1.

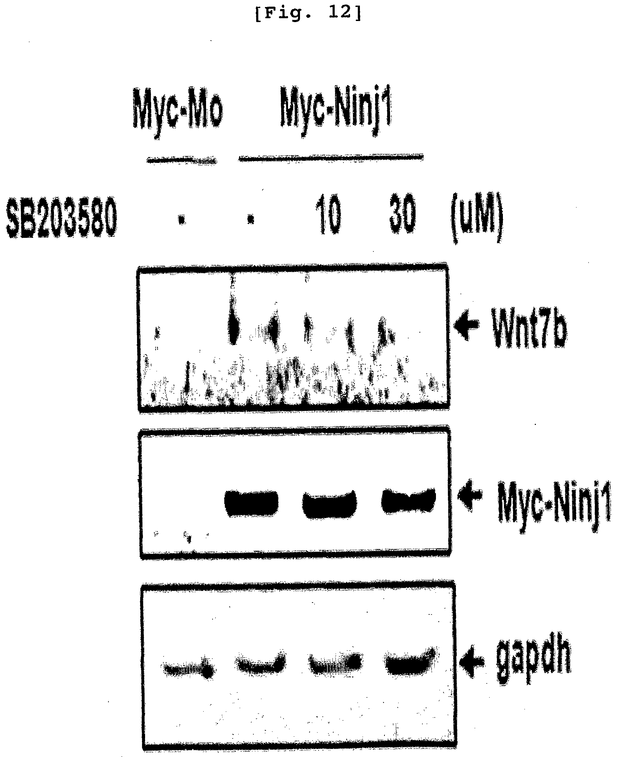

Figure 12 is a diagram illustrating the Wnt7b expression according to the inhibition of p38 expression. BV2 cells expressing Myc-mock and BV2 cells expressing Myc-Ninjurin 1 were treated with SB203580, the p38 inhibitor, at different concentrations, followed by RT-PCR examining the expression of Wnt7b.

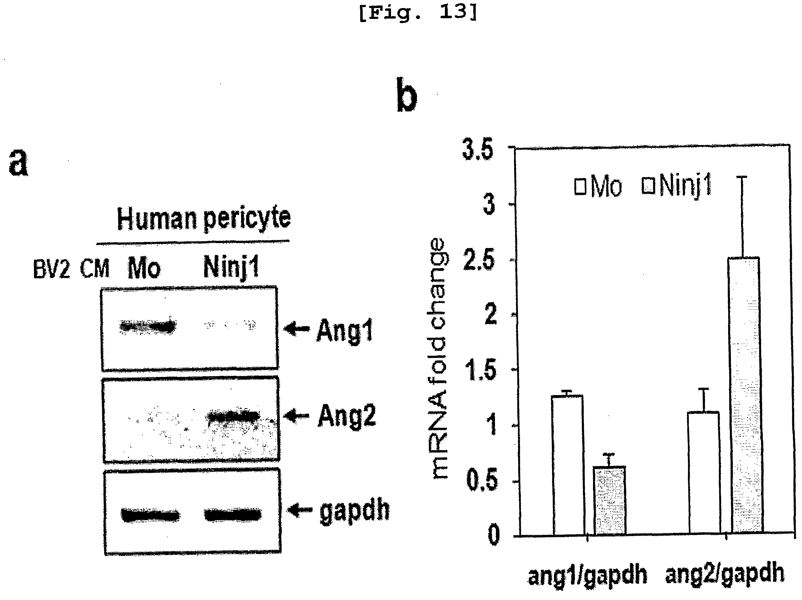

Figure 13 is a diagram illustrating the expressions of Ang1 and Ang2 over Ninjurin 1 expression. Pericytes were treated with culture solutions of BV2 cells expressing Myc-mock and BV2 cells expressing Myc-Ninjurin 1, followed by RT-PCR (left) and real-time PCR (right) to investigate Ang1 and Ang2 expressions.
Figure 14 is a diagram illustrating the levels of Wnt7b and Ninjurin 1 according to the concentrations of Ang1 and Ang2 in rodent originated macrophages (Raw264.7, BV2) and BV2 cells. RT-PCR was performed to quantify Wnt7b and Ninjurin 1 affected by different concentrations of recombinant human Ang1 (rh-Ang1) (left) and by different concentrations of recombinant human Ang2 (rh-Ang2) (right).
Figure 15 is a set of diagrams illustrating the caspase 3 digestion and apoptosis by Ninjurin 1 in HUVEC cells in the presence of rh-Ang2: (a) diagram illustrating the result of Western blotting examining caspase 3 digestion in HUVEC cells treated with culture solutions of BV2 cells expressing Myc-mock and BV2 cells expressing Myc-Ninjurin 1; and (b) diagram illustrating the result of immunostaining showing caspase 3 (red), TUNEL (green) and nucleus (blue) in HUVEC cells treated with culture solutions of BV2 cells expressing Myc-mock and BV2 cells expressing Myc-Ninjurin 1.
Figure 16 is a set of diagrams illustrating the Ninjurin 1 expression under the inflammation condition induced by intraperitoneal injection of LPS into Sprague-Dawley (SD) line SPF rat: (a) diagram illustrating the result of immunostaining of the rat oculus with Iba-1 (green). The rat was administered with LPS to induce systemic inflammation; and (b) diagram illustrating the result of immunostaining of vitreous of the oculus with Iba-1, in which Ninjurin 1 was stained red.
Figure 17 is a set of diagrams illustrating the Ninjurin 1 expression and iNOS expression, the index of inflammation, in BV2 cells treated with LPS: (a) diagram illustrating the iNOS expression examined by Western blotting with BV2 cells treated with different concentrations of LPS; and (b) diagram illustrating the Ninjurin 1 expression examined by RT-PCR and Western blotting with BV2 cells treated with different concentrations of LPS.

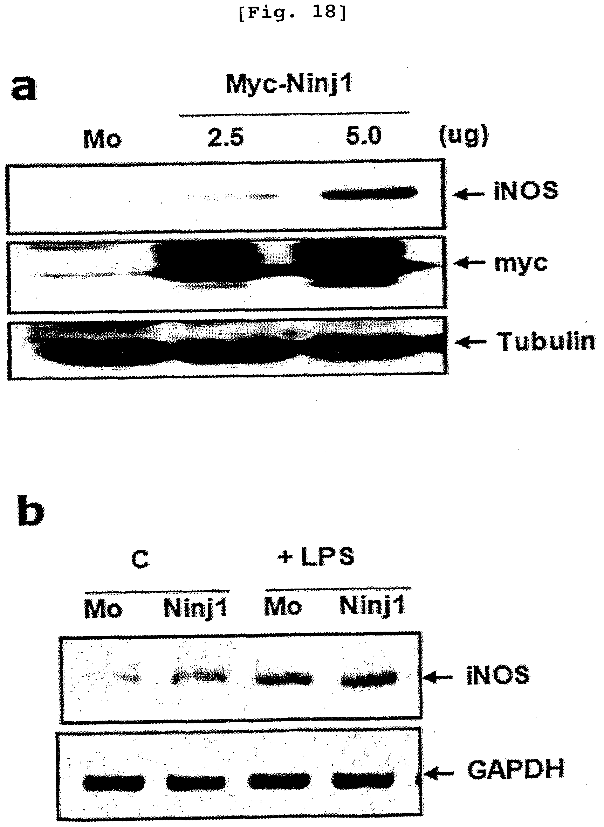

Figure 18 is a set of diagrams illustrating the iNOS expression over Ninjurin 1 expression: (a) diagram illustrating the result of Western blotting examining iNOS expressions in BV2 cells expressing Myc-mock and BV2 cells expressing Myc-Ninjurin 1; and (b) diagram illustrating the result of RT-PCR examining iNOS expressions in BV2 cells expressing Myc-mock and BV2 cells expressing Myc-Ninjurin 1.

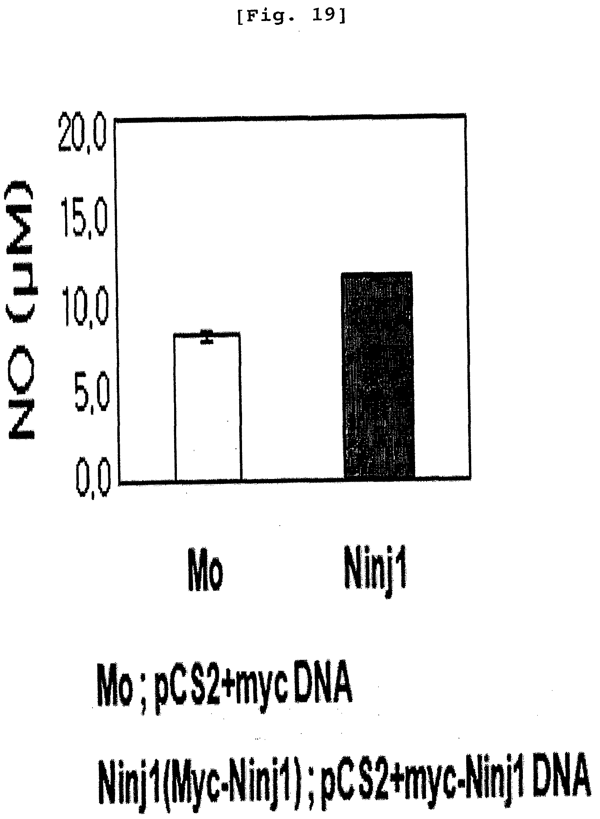

Figure 19 is a diagram illustrating the generation of NO, the inflammatory mediator, over Ninjurin 1 expression. NO levels in BV2 cells expressing Myc-mock and BV2 cells expressing Myc-Ninjurin 1 were quantified by using Griess reagent.

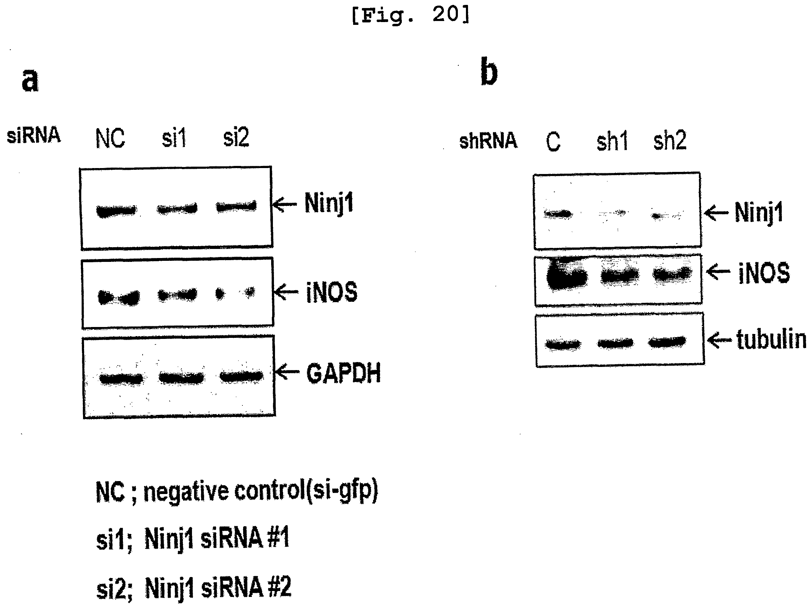

Figure 20 is a set of diagrams illustrating the iNOS expression according to the decrease of Ninjurin 1 expression: (a) diagram illustrating the result of RT-PCR examining iNOS expression when Ninjurin 1 expression was inhibited by siRNA in BV2 cells expressing Ninjurin 1; and (b) diagram illustrating the result of RT-PCR examining iNOS expression when Ninjurin 1 expression was inhibited by shRNA in BV2 cells expressing Ninjurin 1.

### [Mode for Invention]

Practical and presently preferred embodiments of the present invention are illustrative as shown in the following Examples.

### Example 1: Preparation of test animals and conditions thereof

Specific pathogen-free (SPF) pregnant Sprague-Dawley (SD) line rats and male ICR mice/SD rats were purchased from Samtaco BioKorea Co., and then maintained in an animal facility at College of Pharmacy Seoul National University under germ-free conditions during the whole experimental period. All the animal tests were approved by Institute of Laboratory Animal Resources Seoul National University (http://ilar.snu.ac.kr).

### Example 2: Cell culture and preparation of cell lysate and cell culture solution and conditions thereof

Primary cultured human microvascular pericytes (Applied Cell Biology Research Institute) were cultured in Dulbecco's modified Eagle's medium (DMEM); Raw 264.7 cells (TIB-71™) and BV2 cells were cultured in DMEM; and HUVEC cells were cultured in M199 supplemented with 20% FBS (Invitrogen, Grand Island, NY). Those cells were cultured at 37°C in humid air composed of 95% O₂ and 5% CO₂. Raw cells and BV2 cells were transformed by using Lipofectamine Plus Reagent (Invitrogen). Cell lysate was prepared by using lysis buffer (40 mM Tris-Cl pH 8.4, 10 mM EDTA, 120 mM NaCl, 0.1% NP-40). To prepare culture solution to treat human microvascular pericytes, transformed macrophages were cultured in serum(FBS)-free DMEM for 24 hours, which were then filtered with 0.22 µm filter paper (Millipore), followed by 10 fold concentration using centrifugation filtering tube (Millipore). For the chemical treatment, cells were cultured at the density of 60-70% and then treated with Ang1 and Ang2 (500-1000 ng/ml) independently or together in serum free condition for 14-16 hours. To understand signal transduction pathway, cells were transformed with Mock or Ninjurin 1 cDNA. Serum was depleted for 14-16 hours and then the cells were treated with SB203580 (Sigma), the p38 inhibitor, for 24 hours.

### Example 3: Construction of Ninjurin 1 protein expression vector

Wild-type mouse Ninjurin 1 cDNA was synthesized from NIH-3T3 fibroblasts by RT-PCR. At this time, the primer represented by SEQ. ID. NO: 2 (5'-GGGAATTCCATGGAGTCGGGCACTGAGGA-3', upstream containing EcoRI restriction enzyme site) and the primer represented by SEQ. ID. NO: 3 (5'-CTCCTCGAGTTCTACTGCCGGGGCGCCACGT-3', downstream containing Xho I restriction enzyme site) were used. The synthesized cDNA was inserted in pCS2+ vector (labeled with Myc) to express the cDNA in mammalian cells.

### Experimental Example 1: Ninjurin 1 expression over the time during ocular development

The present inventors sacrificed those mice at 2 days (P2), at 3 days (P3), at 5 days (P5), at 8 days (P8), and at 14 days (P14) and adult mice prepared in Example 1 and extracted their oculus, followed by GS-lectin immunostaining to observe vitreous and incipient retinal vessel development.

Particularly, immunostaining was performed as follows. The antibodies used herein were Ninjurin 1 (1:500, provided from Dr. J. Milbrandt), VE-cadherin, GS-lectin (1:500, Santa Cruz), Iba-1 (1:500, Wako), NG2 (1:300, Chemicon) and cleaved-caspase3 (1:500, Cell Signaling). Nuclei were stained with DAPI and propidium iodide (Molecular Probes). Tissues and cells were reacted with the said primary antibody, followed by reaction with secondary antibody exemplified by Alexa488 conjugated IgG or Alexa546 conjugated IgG (Molecular Probes). Images were obtained by using Axiovert M200 microscope (Zeiss, Oberkochen, Germany), followed by analysis with NIH-image J program. Immunostaining was further performed under the same conditions.

Ninjurin 1 protein expression in each oculus of mice having different days was investigated by Western blotting.

Particularly, Western blotting was performed as follows. At this time, Ninjurin 1 (BD Pharminogen), c-Myc (Santa Cruz), α-tubulin (BioGenex), caspase-3 (Cell signaling), iNOS (Santa Cruz) specific primary antibodies were used. Anti-mouse/rabbit horseradish peroxidase conjugated secondary antibody was purchased from Pierce Chemical Co. Color development was performed by using ECL Plus reagent (Amersham Biosciences), followed by detection with LAS-3000 (Fujifilm). Recombinant Ang1 and Ang2 were purchased from R&D Systems INC. Ponceau S solution was purchased from Sigma. Western blotting was performed under the same conditions.

Analysis of results and statistics were performed as follows. Band strength was quantified by using ImageJ (http://rsb.info.nih.gov/ij/), for which stained gapdh, alpha-tubulin or ponceau S band strength was used as standard. The result was presented as mean value ± standard deviation after being converted as relative percentage. The value of the protein showing the highest strength was considered as 100%. Comparison of statistics between two groups was performed by using Student's t-test. When P<0.05, it was judged as statistically significant. Analysis of results and statistics were performed under the same conditions.

Ninjurin 1 and propidium iodide were double stained in the cross-section oculus extracted from mice at 1 day (P1), at 5 days (P5) and at 14 days (P14). Ninjurin 1 and VE-cadherin in the oculus of the mouse at 5 days (P5) were double immuno-stained, followed by observation under fluorescent microscope.

As a result, as shown in Figure 1 and Figure 2, as ocular development progressed, density of blood vessel was reduced. Ninjurin 1 protein expression was gradually increased up to 5 days from birth and the highest Ninjurin 1 expression was observed in mice at 3 days and at 5 days, but thereafter the expression was gradually decreased (Figure 1 and Figure 2). The above results indicate that Ninjurin 1 protein is most expressed in the early stage of ocular development.

### Experimental Example 2: Ninjurin 1 expression specific in macrophages around blood vessels

The present inventors performed double immunostaining of GS-lectin and Ninjurin 1, Ninjurin 1 and ConA, Ninjurin 1 and F4/80, Ninjurin 1 and Iba-I, and Ninjurin 1 and NG2 in the cross-section oculus of the mouse at 5 days (P5). Then, expressions of those proteins were observed under fluorescent microscope.

Hyaloid vessels and structures were eliminated from the oculus using 5% gelatin and then GS-lectin and Ninjurin 1 in the whole mount oculus were immuno-stained, followed by observation under fluorescent microscope.

As a result, as shown in Figure 3 and Figure 4, Ninjurin 1 was expressed in macrophages around blood vessels but not in parenchymal microglia (Figure 3 and Figure 4). Therefore, Ninjurin 1 protein was confirmed to be expressed specifically in macrophages around blood vessels.

### Experimental Example 3: Effect of Ninjurin 1 on apoptosis of vascular endothelial cells

GS-lectin and Ninjurin 1 in vitreous of the mouse at 8 days (P8) were stained, followed by observation under fluorescent microscope.

Vitreous bodies of mice at 6 days (P6) and at 11 days (p11) were observed as the controls and oculus of mice at 6 days (p6) and at 11 days (p11) neutralized with a Ninjurin 1 antibody were observed as the experimental group under immunofluorescent microscope.

Particularly, to block Ninjurin 1 by using an antibody, 1 mg/kg of Ninjurin 1 (BD) mouse neutralizing antibody or 1 mg/kg of mouse isotype control antibody (Santa Cruz) were intraperitoneally injected into mice at day 1 (P1). The mice were sacrificed at day 6 (P6) and at day 11 (P11). Blood vessels and macrophages in oculus were stained by using GS-lectin.

As a result, as shown in Figure 5 and Figure 6, macrophages expressing Ninjurin 1 were adhered on TUNEL positive vascular endothelial cells (proceeded to apoptosis) and the number of vascular endothelial cells was higher in the mouse group neutralized with a Ninjurin 1 antibody than in the control group (Figure 5 and Figure 6). From the above results, it was confirmed that Ninjurin 1 affected apoptosis of vitreous vascular endothelial cells and in the meantime Ninjurin 1 neutralization by a Ninjurin 1 antibody resulted in the decrease of vitreous vascular endothelial cells.

### Experimental Example 4: Effect of Ninjurin 1 on cell-cell adhesion and cell-matrix adhesion

The present inventors investigated cell adhesion by using BV2 cells transformed with pCS2+-Ninjurin 1 or pCS2+-Mock.

Particularly, to investigate cell-cell adhesion, BV2 cells were stained with Hoechst (H33342) for 10 minutes, followed by washing with DMEM. The cells were detached by using trypsin/EDTA and those cells were distributed together with a neutralizing antibody in a 96-well plate (black bottom) on which a single layer of mouse brain microvascular endothelial cells was coated. The cells were lysed with 0.2% NP-40 and fluorescence of the lysate was measured at 340 nm by ELISA. To investigate cell-matrix adhesion, BV2 cells were loaded in a well plate coated with matrixes such as fibronectin (FN, Invitrogen), type I/IV collagen (col.I/col.IV, BD), gelatin (Sigma) and vitronectin (Vit, Invitrogen). After reaction for 15 minutes, the BV2 cells were washed with PBS twice. The adhered cells were stained with crystal violet, followed by washing twice again. The cells were lysed with 0.2% NP-40 and fluorescence of the lysate was measured at 590 nm by ELISA. At last, the cells transformed with Ninjurin 1 and the cells not-transformed were mixed, followed by investigation of aggregate formation therein according to the conventional method (Araki T, et al., Neuron. 1996 Aug; 17(2): 353-61).

As a result, as shown in Figure 7, Ninjurin 1 expression increased macrophage-vascular endothelial cell adhesion. And, cell-matrix adhesion in BV2 cells expressing Ninjurin 1 was greater than in BV2 cells expressing mock. When BV2 cells were neutralized with a Ninjurin 1 antibody, cell-matrix adhesion capacity of the cells was reduced (Figure 7). As shown in Figure 8, BV2 cells expressing Ninjurin 1 formed an aggregate around vascular cells, that is the cells were growing with forming an aggregate (Figure 8).

Therefore, it was confirmed that Ninjurin 1 induced cell-cell aggregation and accelerated cell-matrix adhesion. Experimental Example 5: Effect of Ninjurin 1 on Wnt-Ang signal transduction system

The present inventors performed RT-PCR and real-time PCR to investigate Wnt7b expression in BV2 cells transformed with Myc-mock or Myc-Ninjurin 1 labeled DNA and Wnt7b expression in cells in which Ninjurin 1 expression was inhibited by using siRNA.

Particularly, RT-PCR and real time PCR were performed as follows: Transformed BV2 cells were cultured in DMEM supplemented with 1% serum for 2 days, and then RNA was extracted by using Trizol reagent (Invitrogen). Following primers were used for RT-PCR: Gapdh: forward primer 5'-ACCACAGTCCATGCCATCAC-3' (SEQ. ID. NO: 4), reverse primer 5'-TCCACCACCCTGTTGCTGTA-3' (SEQ. ID. NO: 5); Ninjurin 1: forward primer 5'-GAGTCGGGCACTGAGGA-3' (SEQ. ID. NO: 6), reverse primer: 5'-GTTGCAGGGGTCTGGTCA-3' (SEQ. ID. NO: 7); Ang1: forward primer 5'-AGGCTTGGTTTCTCGTCAGA-3' (SEQ. ID. NO: 8), reverse primer: 5'-TCTGCACAGTCTCGAAATGG-3' (SEQ. ID. NO: 9); Ang2: forward primer 5'-GCTGCTGGTTTATTACTGAAGAA-3' (SEQ. ID. NO: 10), reverse primer: 5'-TCAGGTGGACTGGGATGTTTAG-3' (SEQ. ID. NO: 11); Wnt7b: forward primer 5'-AAGAACTCCGAGTAGGGAGTCG-3' (SEQ. ID. NO: 12), reverse primer: 5'-TGCGTTGTACTTCTCCTTGAGC-3' (SEQ. ID. NO: 13); Wnt7b: 2^{nd} round forward primer 5'-CCGAGTAGGGAGTCGAGAGG-3' (SEQ. ID. NO: 14), reverse primer: 5'-CACACCGTGACACTTACATTCC-3' (SEQ. ID. NO: 15). The PCR products were separated on 1.2% agarose gel containing EtBr (ethidium bromide), followed by analysis by digital imaging. Primers used for real-time PCR were as follows: Ang2: forward primer 5'-TGTGATCTTGTCTTGGCCGC-3' (SEQ. ID. NO: 16), reverse primer: 5'-AGAGGGAGTGTTCCAAGAAGC-3' (SEQ. ID. NO: 17); Gapdh, Ang1 and Wnt7: same primers as used for RT-PCR were used. Conditions for RT-PCR and real-time PCR were the same.

Wnt7b expression in BV2 cells transformed with Myc-mock or Myc-Ninjurin 1 labeled DNA was observed under immunofluorescent microscope.

As a result, as shown in Figure 9 and Figure 10, Wnt7b expression was increased in BV2 cells expressing Ninjurin 1, compared with in BV2 cells expressing mock. In the meantime, when Ninjurin 1 expression was inhibited, Wnt7b expression was reduced (Figure 9 and Figure 10).

To investigate the effect of Ninjurin 1 on the expressions of p38, MAPK (p44/p42) and JNK (p54/46), Western blotting was performed with the lysate of BV2 cells transformed with Myc-mock or Myc-Ninjurin 1 labeled DNA. The BV2 cells transformed with Myc-mock or Myc-Ninjurin 1 labeled DNA were treated with SB203580, the p38 inhibitor, at different concentrations (0, 10, and 30 uM), followed by investigation of Wnt7b expression.

As a result, as shown in Figure 11, expressions of phosphorylated p38, MAPK (p44/p42) and JNK (p54/46) were increased in BV2 cells expressing Ninjurin 1, compared with in BV2 cells expressing mock (Figure 11). As shown in Figure 12, Wnt7b expression stimulated by Ninjurin 1 was reduced by the p38 inhibitor dose-dependently (Figure 12).

To investigate the effect of Ninjurin 1 on the expressions of Ang1 and Ang2, the culture solution of BV2 cells transformed with Myc-mock or Myc-Ninjurin 1 labeled DNA were treated to human pericytes, followed by RT-PCR and real-time PCR to examine the expressions of angiopoietin-1 (Ang1) and angiopoietin-2 (Ang2).

As shown in Figure 13, Ang1 expression was reduced in pericytes treated with culture solution of BV2 cells expressing Ninjurin 1, compared with in pericytes treated with culture solution of BV2 cells expressing mock, but Ang2 expression was increased in pericytes treated with culture solution of BV2 cells expressing Ninjurin 1, compared with in pericytes treated with culture solution of BV2 cells expressing mock (Figure 13).

To investigate interaction among Wnt7b, Ninjurin 1, Ang1 and Ang2, Wnt7b and Ninjurin 1 were quantified by RT-PCR in the presence of recombinant human Ang1 (rh-Ang1) and rh-Ang2 at different concentrations (0, 1 and 2.5 ug/ml) in rodent originated macrophages (Raw264.7, BV2).

As shown in Figure 14, Wnt7b and Ninjurin 1 expressions were reduced by rh-Ang1 dose-dependently, but increased by rh-Ang2 dose-dependently (Figure 14).

HUVEC cells were treated with culture solution of BV2 cells transformed with Myc-mock or Myc-Ninjurin 1 labeled DNA in the presence of rh-Ang2 for 24 hours. Then, caspase 3 digestion in HUVEC cells was confirmed by Western blotting. And the caspase 3 digestion and TUNEL were confirmed by immunofluorescence staining.

As shown in Figure 15, caspase 3 digestion and apoptosis were increased in HUVEC cells treated with culture solution of BV2 cells expressing Ninjurin 1, compared with in HUVEC cells treated with culture solution of BV2 cells expressing mock in the presence of rh-Ang2 (Figure 15).

Therefore, it was confirmed that Ninjurin 1 expression increases Wnt7b expression, reduces Ang1 expression but increases Ang2 expression, and activates p38, MAPK (p44/42) and JNK (p54/46). That is, Ninjurin 1 regulates Wnt-Ang signal transduction system and causes apoptosis of vascular endothelial cells by Ang2.

### Experimental Example 6: Ninjurin 1 expression pattern under inflammatory condition induced by LPS

To induce inflammation *in vivo,* lipopolysaccharide (LPS) (lipopolysaccharides from *Escherichia coli* 0111:B4, Sigma) was intraperitoneally injected into Sprague-Dawley (SD) line SPF rat. Oculus was extracted from the rat, followed by immunofluorescence staining (Ninjurin 1 and Iba-1 were stained) to investigate Ninjurin 1 expression (Ninjurin 1 stained), and inflow and activation of macrophages and microglias (Iba-I stained).

As a result, as shown in Figure 16, the cells expressing Ninjurin 1 were observed in vitreous after LPS injection and inflow of ovoid macrophages was accelerated and at the same time microglias stretching their arms were changed into activated microglias. That is, LPS injection resulted in the increase of inflow and activation of macrophages and microglias and round shaped macrophages expressing Ninjurin 1 were observed in retina (figure 16).

Therefore, it was confirmed that when inflammation was induced by LPS, the number of macrophages expressing Ninjurin 1 were increased and Ninjurin 1 expression was also increased.

### Experimental Example 7: Inflammation related protein expression over Ninjurin 1 expression

To induce inflammation *in vitro,* BV2 cells were treated with LPS at different concentrations (0, 1, and 2.5 ug/ml), followed by Western blotting and RT-PCR to investigate iNOS expression, the index for inflammation, and Ninjurin 1 expression.

As shown in Figure 17, iNOS was up-regulated by LPS injection and Ninjurin 1 expression was also increased by LPS dose-dependently (Figure 17).

To investigate the effect of Ninjurin 1 expression on iNOS expression, Western blotting and RT-PCR were performed to measure iNOS expression in BV2 cells transformed with Myc-mock or Myc-Ninjurin 1 labeled DNA.

As shown in Figure 18, iNOS expression was increased in BV2 cells over-expressing Ninjurin 1, compared in BV2 cells expressing mock. iNOS expression was increased as Ninjurin 1 expression was increased (Figure 18).

To investigate the effect of Ninjurin 1 expression on the generation of NO, the inflammatory mediator, NO generation was measured in BV2 cells transformed with Myc-mock or Myc-Ninjurin 1 labeled DNA.

Particularly, NO generation was calculated by measuring nitrite, the stable reaction product of NO in the cell culture solution, by using Griess reagent. 100 ul of cell culture solution was mixed with 100 ul of Griess reagent [1% sulfanilamide dissolved in 30% acetate and 0.1% N-(1-naphthyl)ehylenediamine dissolved in 60% acetate, 1:1]. 10 minutes later, OD₅₇₀ was measured by ELISA (Ebert S, et al., J Neuroimmunol. 2005 Feb; 159(1-2): 87-96). The acetate, sulfanilamide and N-(1-naphthyl)ehylenediamine were purchased from Sigma.

As shown in Figure 19, NO generation was increased in BV2 cells over-expressing Ninjurin 1, compared in BV2 cells expressing mock (Figure 19).

To investigate the effect of Ninjurin 1 expression inhibition on iNOS expression, siRNA and shRNA were respectively inserted into a vector, which were inserted into BV2 cells. Then, Ninjurin 1 and iNOS expressions in the BV2 cells where Ninjurin 1 expression was inhibited were quantified by Western blotting and RT-PCR.

As a result, as shown in Figure 20, Ninjurin 1 expression was significantly reduced by siRNA and shRNA and accordingly iNOS expression was reduced (Figure 20).

Therefore, it was confirmed that Ninjurin 1 over-expression increased expression of iNOS, the inflammation index and increased generation of NO, the inflammatory mediator. On the other hand, inhibition of Ninjurin 1 expression resulted in the decrease of iNOS expression. The above results suggest that Ninjurin 1 is deeply involved in inflammation reaction.

The Manufacturing Examples of the composition for the present invention are described hereinafter.

### Manufacturing Example 1: Preparation of pharmaceutical formulations

### <1-1> Preparation of powders

| | |
|---|---|
| Kluyveromyces | 2 g |
| Lactose | 1 g |

Powders were prepared by mixing all the above components, which were filled in airtight packs according to the conventional method for preparing powders.

### <1-2> Preparation of tablets

| | |
|---|---|
| Kluyveromyces | 100 mg |
| Corn starch | 100 mag |
| Lactose | 100 mag |
| Magnesium stearate | 2 mg |

Tablets were prepared by mixing all the above components by the conventional method for preparing tablets.

### <1-3> Preparation of capsules

| | |
|---|---|
| Kluyveromyces | 100 mg |
| Corn starch | 100 mg |
| Lactose | 100 mg |
| Magnesium stearate | 2 mg |

Capsules were prepared by mixing all the above components, which were filled in gelatin capsules according to the conventional method for preparing capsules.

### <1-4> Preparation of pills

| | |
|---|---|
| Kluyveromyces | 1 g |
| Lactose | 1.5 g |
| Glycerin | 1 g |
| Xylitol | 0.5 g |

Pills were prepared by mixing all the above components according to the conventional method for preparing pills. Each pill contained 4 g of the mixture.

### <1-5> Preparation of granules

| | |
|---|---|
| Kluyveromyces | 150 mg |
| Soybean extract | 50 mg |
| Glucose | 200 mg |
| Starch | 600 mg |

All the above components were mixed, to which 100 mg of 30% ethanol was added. The mixture was dried at 60°C and the prepared granules were filled in packs.

### [Industrial Applicability]

The present invention can be effectively applied in the development of drugs for diverse diseases caused by over-activation of macrophages induced by over-expression of Ninjurin 1 such as rheumatic arthritis, inflammatory bowel disease, ankylosing spondylitis, psoriasis, atherosclerosis and multiple sclerosis.

Those skilled in the art will appreciate that the conceptions and specific embodiments disclosed in the foregoing description may be readily utilized as a basis for modifying or designing other embodiments of the present invention for carrying out the same purposes.
## Claims

1. A composition composing a Ninjurin 1 expression or activation inhibitor for use in the prevention and treatment of inflammatory disease, wherein the Ninjurin 1 protein expression inhibitor is selected from the group consisting of an antisense nucleotide complementarily binding to Ninjurin 1 mRNA, short interfering RNA complementarily binding to Ninjurin 1 mRNA and short hairpin RNA complementarily binding to Ninjurin 1 mRNA, and wherein the Ninjurin 1 protein activation inhibitor is an antibody binding to Ninjurin 1 protein.

2. The composition for use in the prevention and treatment of inflammatory diseases according to claim 1, wherein the Ninjurin 1 protein has the amino acid sequence represented by SEQ. ID. NO: 1.

3. The composition for use in the prevention and treatment of inflammatory disease according to claim 1, wherein the inflammatory disease is selected from the group consisting of rheumatic arthritis, inflammatory bowel disease, ankylosing spondylitis, psoriasis, atherosclerosis and multiple sclerosis.

4. A use of the Ninjurin 1 expression or activation inhibitor for the production of a composition for preventing and treating inflammatory disease, wherein the Ninjurin 1 protein expression inhibitor is selected from the group consisting of an antisense nucleotide complementarily binding to Ninjurin 1 mRNA, short interfering RNA complementarily binding to Ninjurin 1 mRNA and short hairpin RNA complementarily binding to Ninjurin 1 mRNA, and wherein the Ninjurin 1 protein activation inhibitor is an antibody binding to Ninjurin 1 protein.

5. The use of the Ninjurin 1 expression or activation inhibitor according to claim 4, wherein the Ninjurin 1 protein has the amino acid sequence represented by SEQ. ID. NO: 1.

6. The use of the Ninjurin 1 expression or activation inhibitor according to claim 4, wherein the inflammatory disease is selected from the group consisting of rheumatic arthritis, inflammatory bowel disease, ankylosing spondylitis, psoriasis, atherosclerosis and multiple sclerosis.

7. A screening method of a preventive and therapeutic agent for inflammatory disease comprising the following steps : 1) treating samples to a cell line expressing Ninjurin 1 protein; 2) measuring the expression of Ninjurin 1 protein in the cell line; and 3) selecting a sample that inhibited the expression of Ninjurin 1 protein, compared with the expression level in the control.

8. The screening method of a preventive and therapeutic agent for inflammatory disease according to claim 7, wherein the Ninjurin 1 protein of step 1) has the amino acid sequence represented by SEQ. ID: NO: 1.

9. The screening method of a preventive and therapeutic agent for inflammatory disease according to claim 7, wherein the protein expression of step 2) is measured by one of the methods selected from the group consisting of immunofluorescence method, ELISA, Western blotting, and RT-PCR.

10. The screening method of a preventive and therapeutic agent for inflammatory disease according to claim 7, wherein the inflammatory disease of step 2 is selected from the group consisting of rheumatic arthritis, inflammatory bowel disease, ankylosing spondylitis, psoriasis, atherosclerosis and multiple sclerosis.

## Patentansprüche

1. Zusammensetzung, die einen Ninjurin 1-Expressions-oder Aktivierungshemmstoff umfasst zur Verwendung bei der Vorbeugung und Behandlung von Entzündungserkrankungen, wobei der Ninjurin 1-Proteinexpressionshemmstoff ausgewählt ist aus der Gruppe bestehend aus einem Antisensenukleotid, das komplementär an Ninjurin 1-mRNA bindet, short interfering RNA, die komplementär an Ninjurin 1-mRNA bindet und short hairpin-RNA, die komplementär an Ninjurin 1-mRNA bindet und wobei der Ninjurin 1-Proteinaktivierungshemmstoff ein Antikörper ist, der an Ninjurin 1-Protein bindet.

2. Zusammensetzung zur Verwendung bei der Vorbeugung und Behandlung von Entzündungserkrankungen nach Anspruch 1, wobei das Ninjurin 1-Protein die von SEQ ID NO: 1 dargestellte Aminosäuresequenz aufweist. \

3. Zusammensetzung zur Verwendung bei der Vorbeugung und Behandlung von Entzündungserkrankungen nach Anspruch 1, wobei die Entzündungserkrankung ausgewählt ist aus der Gruppe bestehend aus rheumatischer Arthritis, entzündlicher Darmerkrankung, Morbus Bechterew, Psoriasis, Arteriosklerose und Multipler Sklerose.

4. Verwendung eines Ninjurin 1-Expressions- oder Aktivierungshemmstoffs zur Herstellung einer Zusammensetzung zum Vorbeugen und Behandeln von Entzündungserkrankungen, wobei der Ninjurin 1-Proteinexpressionshemmstoff ausgewählt wird aus der Gruppe bestehend aus einem Antisensenukleotid, das komplementär an Ninjurin 1-mRNA bindet, short interfering RNA, die komplementär an Ninjurin 1-mRNA bindet und short hairpin-RNA, die komplementär an Ninjurin 1-mRNA bindet und wobei der Ninjurin 1-Proteinaktivierungshemmstoff ein Antikörper ist, der an Ninjurin 1-Protein bindet.

5. Verwendung des Ninjurin 1-Expressions-oder Aktivierungshemmstoffs nach Anspruch 4, wobei das Ninjurin 1-Protein die durch SEQ ID NO: 1 dargestellte Aminosäuresequenz aufweist.

6. Verwendung des Ninjurin 1-Expressions-oder Aktivierungshemmstoffs nach Anspruch 4, wobei die Entzündungserkrankung ausgewählt wird aus der Gruppe bestehend aus rheumatischer Arthritis, entzündlicher Darmerkrankung, Morbus Bechterew, Psoriasis, Arteriosklerose und Multipler Sklerose.

7. Screeningverfahren nach einem vorbeugenden und therapeutischen Mittel für Entzündungserkrankungen, das die folgenden Schritte umfasst: 1) Anwenden von Proben auf eine Zelllinie, die Ninjurin 1-Protein exprimiert; 2) Messen der Expression von Ninjurin 1-Protein in der Zelllinie; und 3) Auswählen einer Probe, welche die Expression des Ninjurin 1-Proteins gehemmt hat verglichen mit dem Expressionsniveau in der Kontrolle.

8. Screeningverfahren nach einem vorbeugenden und therapeutischen Mittel für Entzündungserkrankungen nach Anspruch 7, wobei das Ninjurin 1-Protein aus Schritt 1) die durch SEQ ID NO: 1 dargestellte Aminosäuresequenz aufweist.

9. Screeningverfahren nach einem vorbeugenden und therapeutischen Mittel für Entzündungserkrankungen nach Anspruch 7, wobei die Proteinexpression aus Schritt 2) durch eines der Verfahren gemessen wird, die ausgewählt werden aus der Gruppe bestehend aus Immunfluoreszenzverfahren, ELISA, Western Blotting und RT-PCR.

10. Screeningverfahren nach einem vorbeugenden und therapeutischen Mittel für Entzündungserkrankungen nach Anspruch 7, wobei die Entzündungserkrankung aus Schritt 2) ausgewählt wird aus der Gruppe bestehend aus rheumatischer Arthritis, entzündlicher Darmerkrankung, Morbus Bechterew, Psoriasis, Arteriosklerose und Multipler Sklerose.

## Revendications

1. Composition comprenant un inhibiteur de l'expression ou de l'activation de la Ninjurine 1 pour son utilisation dans la prévention et le traitement d'une maladie inflammatoire, dans laquelle l'inhibiteur de l'expression de la protéine Ninjurine 1 est sélectionné dans le groupe constitué d'un nucléotide antisens se liant de façon complémentaire à l'ARNm de la Ninjurine 1, d'un petit ARN interférent se liant de façon complémentaire à l'ARNm de la Ninjurine 1 et d'un petit ARN en épingle à cheveux se liant de façon complémentaire à l'ARNm de la Ninjurine 1, et dans lequel l'inhibiteur de l'activation de la protéine Ninjurine 1 est un anticorps se liant à la protéine Ninjurine 1.

2. Composition pour son utilisation dans la prévention et le traitement de maladies inflammatoires selon la revendication 1, dans laquelle la protéine Ninjurine 1 a la séquence d'acides aminés représentée par SEQ ID NO : 1.

3. Composition pour son utilisation dans la prévention et le traitement d'une maladie inflammatoire selon la revendication 1, dans laquelle la maladie inflammatoire est sélectionnée dans le groupe constitué de la polyarthrite rhumatoïde, d'une affection abdominale inflammatoire, de la spondylarthrite ankylosante, du psoriasis, de l'athérosclérose et de la sclérose en plaques.

4. Utilisation d'un inhibiteur de l'activation ou de l'expression de la Ninjurine 1 pour la production d'une composition pour la prévention et le traitement d'une maladie inflammatoire, dans laquelle l'inhibiteur de l'expression de la protéine Ninjurine 1 est sélectionné dans le groupe constitué d'un nucléotide antisens se liant de façon complémentaire à l'ARNm de la Ninjurine 1, d'un petit ARN interférent se liant de façon complémentaire à l'ARNm de la Ninjurine 1 et d'un petit ARN en épingle à cheveux se liant de façon complémentaire à l'ARNm de la Ninjurine 1, et dans laquelle l'inhibiteur de l'activation de la protéine Ninjurine 1 est un anticorps se liant à la protéine Ninjurine 1.

5. Utilisation de l'inhibiteur de l'expression ou de l'activation de la Ninjurine 1 selon la revendication 4, dans laquelle la protéine Ninjurine 1 a la séquence d'acides aminés représentée par SEQ ID NO : 1.

6. Utilisation de l'inhibiteur de l'expression ou de l'activation de la Ninjurine 1 selon la revendication 4, dans laquelle la maladie inflammatoire est sélectionnée dans le groupe constitué de la polyarthrite rhumatoïde, d'une affection abdominale inflammatoire, de la spondylarthrite ankylosante, du psoriasis, de l'athérosclérose et de la sclérose en plaques.

7. Procédé de criblage d'un agent prophylactique et thérapeutique pour une maladie inflammatoire comprenant les étapes suivantes: 1) administration d'échantillons à une lignée cellulaire exprimant la protéine Ninjurine 1 ; 2) mesure de l'expression de la protéine Ninjurine 1 dans la lignée cellulaire; et 3) sélection d'un échantillon qui a inhibé l'expression de la protéine Ninjurine 1, comparé au niveau d'expression dans le témoin.

8. Procédé de criblage d'un agent préventif et thérapeutique pour une maladie inflammatoire selon la revendication 7, dans lequel la protéine Ninjurine 1 de l'étape 1) a la séquence d'acides aminés représentée par SEQ ID NO : 1.

9. Procédé de criblage d'un agent préventif et thérapeutique pour une maladie inflammatoire selon la revendication 7, dans lequel l'expression de la protéine de l'étape 2) est mesurée par un des procédés sélectionnés dans le groupe constitué d'un procédé d'immunofluorescence, d'un ELISA, d'un transfert de type Western, et d'une PCR-RT.

10. Procédé de criblage d'un agent préventif et thérapeutique pour une maladie inflammatoire selon la revendication 7, dans lequel la maladie inflammatoire de l'étape 2 est sélectionnée dans le groupe constitué de la polyarthrite rhumatoïde, d'une affection abdominale inflammatoire, de la spondylarthrite ankylosante, du psoriasis, de l'athérosclérose et de la sclérose en plaques.
